# EUROPÄISCHE PATENTSCHRIFT

(11) **EP 2 084 724 B1**
(45) Veröffentlichungstag und Bekanntmachung des Hinweises auf die Patenterteilung: **09.02.2011**
(21) Anmeldenummer: 07866223.6
(22) Anmeldetag: 07.12.2007
(51) Int. Cl.: H01F 38/14

(54) **INDUKTIVER DREHÜBERTRAGER**
INDUCTIVE ROTARY JOINT
TRANSFORMATEUR TOURNANT INDUCTIF

(30) Priorität: 07.11.2006 DE 102006052685
(43) Veröffentlichungstag der Anmeldung: 05.08.2009
(73) Patentinhaber: Schleifring und Apparatebau GmbH, 82256 Fürstenfeldbruck (DE)
(72) Erfinder: KRUMME, Nils, 82340 Feldafing (DE); LOHR, Georg, 82223 Eichenau (DE); WEITHMANN, Herbert, 80999 München (DE); BLEY, Michael, 82216 Maisach (DE)
(74) Vertreter: Lohr, Georg
(86) Internationale Anmeldenummer: PCT/EP2007/009631
(87) Internationale Veröffentlichungsnummer: WO 2008/055664

(56) Entgegenhaltungen:
- WO-A-98/32217
- WO-A-03/085797
- DE-A1- 10 241 581
- US-A1- 2004 218 406

## Beschreibung

### Technisches Gebiet

Die Erfindung betrifft einen induktiven Drehübertrager, zur Übertragung elektrischer Leistung zwischen zwei gegeneinander drehbaren Einheiten, insbesondere zum Einsatz in Computertomographen.

### Stand der Technik

Kontaktlose, induktive Drehübertrager sind ein vorteilhafter Ersatz für die bekannten mechanischen Schleifringe zur Übertragung elektrischer Energie. Bei der induktiven Übertragungstechnik erfolgt die Verkoppelung zwischen drehbaren Einheiten berührungslos durch magnetische Felder. Dies hat gegenüber mechanischen Schleifringen den Vorteil, dass Drehmoment, Verschleiß und somit auch der Wartungsaufwand minimiert wird. Weiterhin wird das Umfeld der Drehübertrager nicht durch Kohlestaub verschmutzt.

Induktive Drehübertrager weisen an jeder der drehbaren Einheiten mindestens eine Wicklung auf. Weiterhin kann sowohl am Rotor als auch am Stator oder auch an beiden Teilen ein Eisenkern oder Ferritkern zur Steuerung des Magnetfeldes vorgesehen sein. Ein Wechselsignal wird in einer Wicklung eines der Teile eingespeist und in einer anderen Wicklung des anderen Teils abgegriffen und einer Last zugeführt. Ein solcher Drehübertrager ist beispielsweise in der DE 29580172 U1 offenbart.

Bei einem galvanisch gekoppelten Schleifring kann auf einfache Weise beispielsweise eine konstante Spannung einer Spannungsquelle in den Schleifring eingespeist und auf der anderen Seite von der Last abgegriffen werden. Aufgrund der niederohmigen galvanischen Verbindung durch den Schleifring entspricht die Ausgangspannung bis auf geringe Abweichungen der Eingangspannung. Durch den geringen Widerstand des Schleifrings ergibt sich ein geringfügiger und meist vernachlässigbarer Spannungsabfall in Abhängigkeit vom Laststrom.

Bei induktiv gekoppelten Drehübertragern befindet sich im Übertrager-Ersatzschaltbild der Anordnung eine Streuinduktivität als Serieninduktivität zwischen der Eingangseite und der Lastseite. Diese Streuinduktivität ist abhängig von der eigenen Induktivität des Übertragers und insbesondere vom Koppelfaktor. Gerade bei induktiven Drehübertragern mit großen Abmessungen lässt sich oftmals nur ein geringer Koppelfaktor realisieren, der zudem häufig noch mit der relativen Position der drehbaren Einheiten zueinander schwankt. So nimmt der Koppelfaktor beispielsweise mit steigendem Luftspalt zwischen den gegeneinander drehbaren Eisenkernen ab. Die Streuinduktivität nimmt dann entsprechend zu. Um nun trotz dieser Streuinduktivität eine höhere Leistung über den Drehübertrager übertragen zu können, wird die Streuinduktivität wie eine diskrete Induktivität in geeigneten Schaltungen eingesetzt. Anwendungen wären beispielsweise als Speicherinduktivität oder auch als Resonanzinduktivität. Im Falle einer Resonanzinduktivität kann die Induktivität beispielsweise mit einer Serienkapazität zu einem Serienresonanzkreis oder mit einer Parallelkapazität zu einem Parallelresonanzkreis ergänzt werden. Selbstverständlich können auch komplexere Filterstrukturen realisiert werden. Ein solcher Drehübertrager mit Resonanzkreisen ist beispielsweise in der EP 0 953 225 A offenbart. Problematisch bei derartigen Schaltungen ist, dass immer eine Messeinrichtung auf der Ausgangsseite des Drehübertragers benötigt wird. So variiert in den meisten bekannten Schaltungen mit einer Serieninduktivität wenigstens eine, meist auch mehrere der Ausgangs-Kenngrößen, wie Strom, Spannung oder Leistung mit einer Änderung der Lastimpedanz. Während bei einem kontaktierenden Schleifring vorzugsweise die Last mit einer konstanten Spannung gespeist wird, die problemlos über den Schleifring übertragen werden kann und weitgehend lastunabhängig ist, ändern sich bei einem typischen kontaktlosen Drehübertrager mit einer Serieninduktivität Ausgangspannung, Ausgangstrom und entsprechend auch die abgegebene Leistung mit einer Änderung der Lastimpedanz. Weiterhin ändert sich die Serieninduktivität durch mechanische Toleranzen während der Bewegung der Teile gegeneinander. Um hier eine gleichmäßige Versorgung der Ausgangsseite zu erreichen und eine Zerstörung der angeschlossen Komponenten zu verhindern, ist es notwendig, wenigstens eine der elektrischen Kenngrößen an der Ausgangsseite zu regeln. Bei kleinen Leistungen kann ein separater Regler, wie ein Spannungsregler, der beispielsweise als Serienregler oder auch als Schaltregler aufgebaut ist, eingesetzt werden. Bei größeren Leistungen ist auf der Ausgangsseite wenigstens ein Sensor für eine dieser elektrische Größen anzubringen.

Dieser Sensor ermittelt die Größe und signalisiert sie an die Wechselsignalquelle auf der Eingangseite. Durch einen Regelverstärker kann nun auf der Eingangseite eine elektrische Kenngröße, wie beispielsweise Strom, Spannung oder auch die Frequenz so geregelt werden, dass eine Versorgung, beispielsweise mit konstanter Spannung sichergestellt ist. Bei konventionellen Schaltnetzteilen ist eine solche Technik bekannt und wird auch regelmäßig eingesetzt. Bei Drehübertragern besteht das Problem, das die Information des Sensors von der Ausgangsseite auf die Eingangseite, also zwischen zwei gegeneinander drehbaren Einheiten übertragen werden muss. Dies erfordert einen weiteren Drehübertrager in umgekehrter Übertragungsrichtung wie der induktive Leistungsübertrager. Eine Lösung des Problems ist beispielsweise in der DE 29580172 U1 als kapazitives Koppelelement offenbart. Oftmals steht aber kein mechanischer Bauraum für ein derartiges kapazitives Koppelelement zur Verfügung, beziehungsweise ein solches Koppelelement wird zur Datenübertragung für andere Daten, wie beispielsweise im Messdaten benötigt und kann somit nicht für die Regelung eingesetzt werden.

Die hier dargestellten Probleme nehmen mizunehmender Größe des Drehübertragers zu. So können bei kompakten Einheiten mit Durchmessern von wenigen Zentimetern noch präzise Lager mit Toleranzen unter 0,1 mm eingesetzt werden. Damit ist beispielsweise ein präziser Luftspalt mit 0,2 mm und einer Schwankung im Bereich von 0,2 mm bis 0,3 mm realisierbar. Bei großen Einheiten, mit Durchmessern größer einem Meter wie sie beispielsweise in Computertomographen eingesetzt werden, liegen die Toleranzen schon im Bereich von einigen Millimetern, teilweise größer 5 mm. So würde in einem solchen Fall positions- und betriebszustandabhängig der Luftspalt zwischen 1 und 6 mm variieren. Dies führt zu einer wesentlich höheren Streuinduktivität, welche zudem wesentlich stärker schwankt.

In der DE 102004051170 A1 ist ein induktiver Drehübertrager für Computertomographen mit einem rotierenden Transformator offenbart, bei dem sich die Primärseite gegenüber der Sekundärseite dreht.

In der US 2004/0218406 A1 ist eine kontaktlose elektrische Energieübertragung offenbart. Es wird hierbei zur Regelung des Sekundärstroms der Primärstrom des Transformators gemessen.

### Darstellung der Erfindung

Der Erfindung liegt die Aufgabe zugrunde, einen Drehübertrager zur kontaktlosen induktiven Übertragung elektrischer Leistung derart zu gestalten, dass eine Rückkopplung von einem Ausgangsseitigen Sensor für wenigstens eine elektrische Kenngröße zu einer eingangsseitigen Wechselstromquelle nicht mehr benötigt wird.

Erfindungsgemäße Lösungen dieser Aufgabe sind in den unabhängigen Patentansprüchen angegeben. Weiterbildungen der Erfindung sind Gegenstand der abhängigen Ansprüche.

Die erfindungsgemäße Vorrichtung umfasst einen Leistungsgenerator 11 zur Erzeugung einer Wechselspannung oder eines Wechselstromes, alternativ eines pulsierenden Gleichstroms. Weiterhin ist ein Leistungsübertrager vorgesehen, welcher eine Primärseite 12 und eine Sekundärseite 21 umfasst. Die Primärseite 12 wird durch einen Leistungsgenerator gespeist, während die Sekundärseite 21 zur Speisung einer Last 22 dient. Primärseite 12 und Sekundärseite 21 sind gegeneinander drehbar angeordnet. So befindet sich die Primärseite 12 beispielsweise an einem stationären Teil 2 und die Sekundärseite 21 an einem rotierenden beziehungsweise rotierbaren Teil 1. Weiterhin ist entsprechend der Erfindung eine Messeinrichtung 15 vorgesehen, welche wenigstens eine elektrische Kenngröße an der Primärseite 12 des Leistungsübertragers ermittelt. Eine solche Kenngröße kann beispielsweise eine Spannung, wie die Spannung an der Primärseite 12, ein Strom wie der Strom in die Primärseite 12, eine Leistung, wie die in der die Primärseite 12 eingespeiste Leistung oder auch ein Phasenwinkel, wie beispielsweise der Phasenwinkel zwischen Strom und Spannung an der Primärseite 12 sein. Entsprechend der Erfindung können auch weitere elektrische Kenngrößen, wie beispielsweise die spektrale Zusammensetzung ermittelt werden. Weiterhin ist eine Funktionseinheit 17 vorgesehen, welche die wenigstens eine elektrische Kenngröße von der Messeinrichtung 15 weiter auswertet und daraus ein Steuersignal für den Leistungsgenerator 11 erzeugt. Mittels dieses Steuersignals wird der Leistungsgenerator 11 derart gesteuert, dass zumindest eine weitere elektrische Kenngröße an der Last 22 näherungsweise konstant ist. Eine solche weitere elektrische Kenngröße kann beispielsweise eine Spannung, wie die Spannung an der Last 22, ein Strom, wie der Strom in die Last 22, eine Leistung, wie die in die Last 22 abgegebene Leistung, oder auch ein Phasenwinkel, wie beispielsweise der Phasenwinkel zwischen Strom und Spannung an der Last 22 sein. Um nun diese weitere elektrische Kenngröße näherungsweise konstant zu halten, weist die Funktionseinheit 17 ein Modell zur Nachbildung der Übertragungsfunktion, das heißt des Zusammenhangs zwischen der wenigstens einen elektrischen Kenngröße an der Last 23 und der wenigstens einen Kenngröße an der Primärseite 12 auf. Weiterhin weist die Funktionseinheit 17 alle notwendigen Komponenten auf, um einen geschlossenen Regelkreis zu erhalten. Dies bedeutet im Normalfall, dass noch ein Differenzverstärker vorgesehen ist, der das Ausgangssignal des Modells zur Nachbildung der Übertragungsfunktion mit einem vorgegebenen Sollwert vergleicht und diesen dann verstärkt an den Leistungsgenerator 11 weiter leitet. Der Differenzverstärker kann vorteilhafterweise dem Stand der Technik entsprechend als Proportional-, Proportional/Integral- oder auch Proportional/Integral/Differential-Verstärker ausgelegt sein. In dem besonders einfachen Fall wird von der Funktionseinheit 17 nur ein Steuersignal, beispielsweise für die Leistungsabgabe an den Leistungsgenerator 11 signalisiert. Eine höhere Flexibilität kann aber erreicht werden, wenn mehrere Signale, wie beispielsweise die Leistungsabgabe und die Frequenz an den Leistungsgenerator signalisiert werden. Dies erhöht allerdings die Komplexität der gesamten Anordnung und insbesondere der Funktionseinheit 17.

Insbesondere bei den Elementen Leistungsgenerator 11, Messeinrichtung 15 und dem optionalen Messverstärker 16 handelt es sich um funktionale Elemente, wie beispielsweise auch in eine Baugruppe Leistungsgenerator integriert sein können. Anstelle oder zusätzlich zum Messverstärker 15 kann auch noch wahlweise eine analoge und oder digitale Filterung der Messgrößen vorgesehen sein.

Vorteilhafterweise ist das Modell eine Kennlinie oder eine Kennlinienschar. Diese Kennlinie oder Kennlinienschar kann entsprechend der Anzahl der von der Messeinrichtung 15 gelieferten Eingangsparameter und der an den Regelverstärker 14 abgegebenen Ausgangsparameter auch mehrdimensional sein.

In einer besonders vorteilhaften Ausgestaltung der Erfindung erfolgt die Modellierung der Kennlinie aufgrund der Ergebnisse einer Schaltungssimulation, wobei vorzugsweise die Abhängigkeit der Spannung an der Last 22 als Funktion der Schaltfrequenz des Leistungsgenerators 11 und des Stromes in die Primärseite 12 des Leistungsübertragers dargestellt wird.

In einer weiteren vorteilhaften Ausgestaltung der Erfindung bildet das Modell das Verhalten der Anordnung aufgrund eines vereinfachten Ersatzschaltbildes nach. Hierbei werden vorzugsweise nur die resistiven Anteile der Schalttransistoren und Leitungen, die parasitären Anteile der Wicklungen und Induktivitäten sowie bewusst eingebrachter Widerstände berücksichtigt. Ferner wird das induktive Ersatzschaltbild des Leistungsübertragers mit der Primärseite 12 und der Sekundärseite 13 herangezogen. Schließlich wird noch die Kapazität von eventuell vorhandenen Resonanzkondensatoren berücksichtigt.

In einer anderen Ausgestaltung erfolgt die Modellierung aufgrund von wenigstens einer durch Messung gewonnenen Übertragungskennlinie. Es stellt vorteilhafter weise den Zusammenhang der Abhängigkeit der Ausgangspannung von der Schaltfrequenz des Leistungsgenerators 11 und dem in die Primärseite 12 des Leistungsübertragers eingespeisten Strom zu der von der Last 22 aufgenommenen Leistung beziehungsweise dem Widerstand der Last 22 dar.

In einer weiteren Ausgestaltung nähert das Modell zumindest einen Bereich der Übertragungsfunktion der Anordnung als lineare Funktion durch eine Gerade an. Dies ist besonders dann sinnvoll, wenn die Last eine bekannte und vorzugsweise begrenzte Dynamik hat. Ist ein minimaler und ein maximaler Lastwiderstand bekannt, so kann meist die Kennlinie vorzugsweise durch eine gerade in diesem Bereich angenähert und optimiert werden.

Eine andere Ausgestaltung sieht ein Modell in Form einer Nachbildung der Übertragungsfunktion durch analoge, passive Bauteile vor. Diese könnten Bauteile mit entsprechenden elektrischen werden der Bauteile im Leistungspfad zwischen Leistungsgenerator 11 und Last 22 sein, wobei deren Belastbarkeit wesentlich geringer sein kann.

Eine andere Ausgestaltung sieht vor, dass das Modell zumindest einen Bereich der Übertragungsfunktion mittels eines Widerstands- und Diodennetzwerks annähert. Ein solches Netzwerk kann beispielsweise in der Beschaltung eines Operationsverstärkers vorgesehen sein.

Alternativ hierzu ist auch eine Annäherung der Übertragungsfunktion mittels eines logarithmischen Verstärkers möglich.

Vorteilhafterweise wird in das Modell auch wenigstens eine mechanische Größe, wie der Abstand zwischen rotierendem Teil 1 und stationärem Teil 2, die Größe des Luftspalts zwischen der Primärseite 12 des Leistungsübertragers und der Sekundärseite 21 des Leistungsübertragers, oder einer Winkelposition zwischen rotierendem Teil 1 und stationärem Teil 2 mit einbezogen, wobei im Letzteren Falle beispielsweise die Übertragungsfunktion durch Summierung verschiedener mechanischer Parameter und Toleranzen positionsabhängig ist. Das heranziehen einer mechanischen Größe ist besonders sinnvoll, da ein direkter Zusammenhang zwischen der Streuinduktivität und dem Luftspalt, das heißt dem Abstand zwischen der Wicklungen beziehungsweise dem Kern der Primärseite 12 des Leistungsübertragers und der Sekundärseite 21 des Leistungsübertragers besteht. Die Messung einer solchen mechanischen Größe erfolgt vorzugsweise durch einen Weggeber oder Positionencoder, kann aber auch beispielsweise im Falle der Winkelposition durch Zeitmessung der Bewegung ab einem Referenzpunkt ermittelt werden. Ebenso ist eine Messung durch Auswertung der Kapazität zwischen Kondensatorplatten, welche am rotierenden Teil 1 und am stationären Teil 2 angebracht sind, oder der Kapazität zwischen Primärseite und Sekundärseite des Leistungsübertragers möglich. Alternativ kann eine solche mechanische Größe auch durch eine übergeordnete Steuereinheit, welche den kompletten Systemstatus der Anlage, wie beispielsweise eines Computertomographen steuert, signalisiert werden. Weiterhin ist es vorteilhaft, wenn die Funktionseinheit einen digitalen Rechner, vorzugsweise einen digitalen Signalprozessor aufweist.

Wahlweise kann das Modell als mathematisches Modell in der Funktionseinheit 17 hinterlegt sein, so dass die Übertragungsfunktion jeweils zur Laufzeit in Abhängigkeit von den Eingangsparametern berechnet wird.

Wahlweise kann auch das Modell in Form einer Tabelle, mit oder ohne Interpolation, in einem Speicher wie beispielsweise in einem ROM abgelegt sein.

Eine andere vorteilhafte Ausgestaltung sieht vor, dass die Funktionseinheit 17 Mittel zum Einmessen beziehungsweise Einlernen der Modellparameter besitzt. So können beispielsweise die genauen Parameter des Modells in einem speziellen Betriebszustand bei oder vor der Inbetriebnahme der Vorrichtung ermittelt werden. So könnte beispielsweise hilfsweise ein Messkabel oder auch eine digitale Datenstrecke zur Messung der Spannung an der Last 22 zur Funktionseinheit 17 geführt werden, so dass diese für verschiedene Zustände des Leistungsgenerators die jeweiligen Zustände an der Last ermitteln kann. Weiterhin könnten zusätzlich unterschiedliche Lasten zur Ermittlung der Kennlinie anstelle der Last 22 geschaltet werden. Dieses Einmessen kann beispielsweise ohne Ausführen einer Drehbewegung durch Überbrückung des Drehübertragers mit einem Kabel erfolgen. Alternativ kann mit einem Kabel auch eine beschränkte Drehbewegung, beispielsweise um 360° ausgeführt werden, um die Winkelabhängigkeit zu erfassen. Es könnte auch hilfsweise ein weiterer Drehübertrager angebracht werden, um mehrere Umdrehungen oder einen rotierenden Betrieb zu erfassen.

Besonders günstig ist es, wenn bestimmte Betriebszustände, wie Kurzschluss oder Leerlauf zur Ermittlung der Parameter herangezogen werden können. Derartige Betriebszustände ergeben sich beispielsweise beim Hochfahren der Schaltung, sie können jedoch auch gesteuert durch Schalter gezielt herbeigeführt werden (Leerlauf, Kurzschluss). So besteht ein niederohmiger, kurzschlussähnlicher Betriebszustand, wenn die Filterkondensatoren hinter einem Gleichrichter für die Versorgungsspannung der Last 22 noch nicht aufgeladenen sind. Ein hochohmiger Betriebszustand könnte sich einstellen, wenn sich die Last 22 erst mit einer gewissen Zeitverzögerung, oder erst ab einer vorgegebenen Eingangsspannung zuschaltet. Aus diesen Zuständen können weitere Parameter ermittelt werden. Die Ursachen für der Art besondere Betriebszustände können auch gezielt beeinflusst werden, so das diese Betriebszustände für eine längere Zeit, ausreichend zur Messung bestehen. So könnten beispielsweise die Filterkondensatoren vergrößert oder auch eine Zeitverzögerung verlängert werden.

Durch zusätzliche weitere Schutzschaltungen kann die Zuverlässigkeit erhöht werden. So kann beispielsweise eine Spannung wie die Spannung an der Last 22 oder eine Spannung an der Primärseite 12 des Leistungsübertragers überwacht werden. Weiterhin ist eine Abschätzung des Wertes der Spannung an der Last 22 unter Zuhilfenahme der Funktionseinheit 17 möglich. Alternativ hierzu könnte auch ein Signalübertrager 24, 13 für die Spannungsüberwachung eingesetzt werden. Bei Überschreiten eines Grenzwertes kann für den Fall, dass der Leistungsübertrager als Serienresonanzkreis geschaltet ist, ein Kurzschluss der Sekundärseite 21 des Leistungsübertragers erfolgen. Ebenso kann für den Fall, dass der Leistungsübertrager als Parallelresonanzkreises geschaltet ist, die Sekundärseite 21 des Leistungsübertragers von der Last 22 getrennt werden. Besonders günstig ist es in diesem Fall, dass die Last 22 von der Sekundärseite 21 des Leistungsübertragers vollständig getrennt bleibt, bis sich die Kapazitäten in der Anordnung hinreichend entladen haben.

Zur Veranschaulichung des Funktionsprinzips wird hier eine Drehübertragung von einem feststehenden Teil (Stator) auf ein rotierendes Teil (Rotor) erläutert. Ebenso ist auch eine Übertragung zwischen einem rotierenden Teil und einem feststehenden Teil möglich, da dies ohnehin nur eine Frage des Ortsbezugs ist. Weiterhin ist entsprechend der Erfindung auch eine induktive Übertragung zwischen linear gegeneinander beweglichen Teilen realisierbar. Die Erfindung betrifft die induktive Kopplung zwischen gegeneinander beweglichen Teilen in allgemeiner Form und ist unabhängig von der Art der Bewegung dieser Teile gegeneinander. So ist sie nicht nur auf Drehübertragers sondern beispielsweise auch auf lineare Übertrager anwendbar.

Ein erfindungsgemäßer Computertomograph umfasst wenigstens einen induktiven Drehübertrager, wie er oben beschrieben ist.

### Beschreibung der Zeichnungen

Die Erfindung wird nachstehend ohne Beschränkung des allgemeinen Erfindungsgedankens anhand eines Ausführungsbeispiels unter Bezugnahme auf die Zeichnung exemplarisch beschrieben.
Figur 1 zeigt in allgemeiner Form schematisch einen Computertomographen
Figur 2 zeigt schematisch eine Ausführungsform nach dem Stand der Technik
Figur 3 zeigt schematisch eine erfindungsgemäße Ausführungsform eines kontaktlosen Drehübertragers

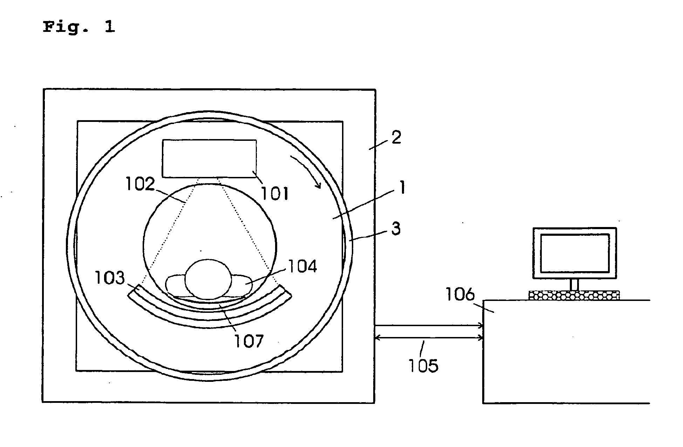

Fig. 1 zeigt beispielhaft eine erfindungsgemäße Vorrichtung. Der Computertomograph (CT-Scanner) besteht aus zwei mechanischen Hauptbestandteilen. Ein stationäres Teil 2 dient als Basis und Träger des ganzen Gerätes, in denen sich das rotierende Teil 1 dreht. Der Patient 104 wird auf einer Liege 107 in der Öffnung des rotierenden Teils positioniert. Zur Abtastung des Patienten mittels Röntgenstrahlen 102 ist eine Röntgenröhre 101 sowie ein dieser gegenüberliegend angeordneter Detektor 103 vorgesehen. Röntgenröhre 101 und Detektor 103 sind auf dem rotierenden Teil 1 drehbar angeordnet. Ein Drehübertrager 3 dient zur elektrischen Verbindung zwischen dem rotierenden Teil 1 und dem stationären Teil 2. Hierbei werden einerseits die hohe elektrische Leistung zur Speisung der Röntgenröhre 101 in Richtung des rotierenden Teils 1 und gleichzeitig die Rohdaten des Bildes in der entgegengesetzten Richtung übertragen. Parallel hierzu ist eine Kommunikation von Steuerinformationen in beiden Richtungen vorgesehen. Eine Auswerte- und Steuereinheit 106 dient zur Bedienung des Computertomographen sowie zur Anzeige der erzeugten Bilder. Die Kommunikation mit dem Computertomographen erfolgt über eine bidirektionale Verbindung 105.

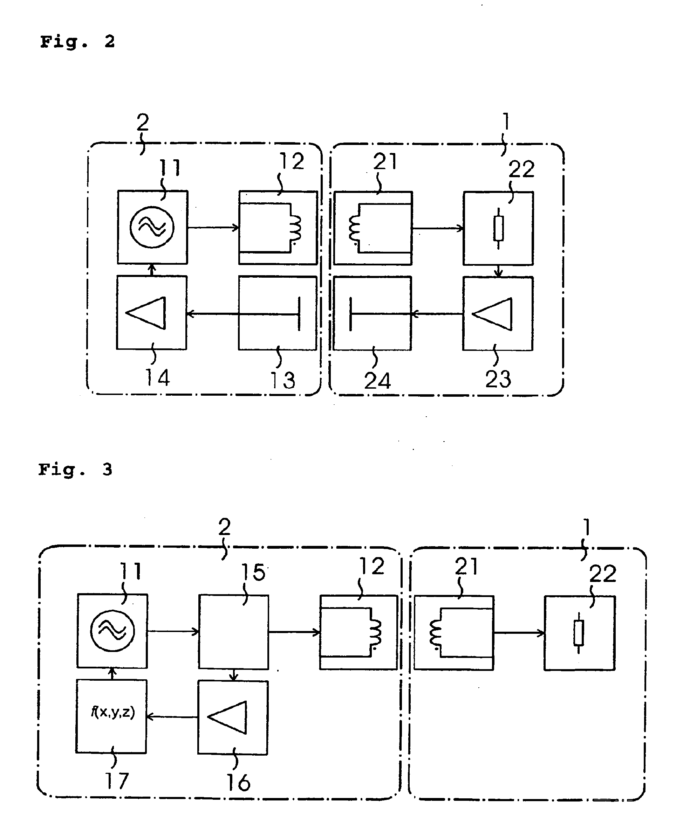

Fig. 2 zeigt schematisch den aus DE 10 2004 051 170 A1 bekannten kontaktlosen Drehübertrager. Es wird Energie von einem stationären Teil 2 zu einem rotierenden Teil 1 übertragen. Im stationären Teil 2 befinden sich ein Leistungsgenerator 11, der die Primärseite eines Leistungsübertragers 12 mit einem Wechselsignal oder einem pulsierenden Gleichsignal speist. Der Leistungsgenerator ist vorzugsweise ein Schaltverstärker beziehungsweise eine Schaltstufe. Diese weist vorzugsweise eine Halbbrücken- oder Vollbrückenschaltung, vorzugsweise mit IGBTs oder MOSFets auf. Die Versorgung dieser Stufe erfolgt vorzugsweise aus dem Wechselspannungsnetz, beispielsweise über einen Gleichrichter, bevorzugt über eine Leistungsfaktorkorrekturschaltung.

Die Primärseite 12 des Leistungsübertragers ist magnetisch mit der Sekundärseite 21 des Leistungsübertragers zur Speisung der Last 22 verkoppelt. Ein Messverstärker ermittelt vorbestimmte Messgrößen, die die Last beziehungsweise einen Betriebszustand der Last charakterisieren. Eine solche Messgröße ist beispielsweise die an der Last anliegende Spannung oder der durch die Last fließende Strom. Typischerweise wird eine Last 22 mit Gleichstrom versorgt. Aus diesem Grunde ist zwischen Last 22 und der Sekundärseite 21 des Leistungsübertragers ein Gleichrichter mit optionaler Glättungs- und optionaler Regelschaltung vorgesehen. In diesem Falle wäre die Messgröße eine Gleichspannung an der Last oder ein Gleichstrom durch die Last.

Das Ausgangsignal des Messverstärkers 23 wird mittels eines Signalübertragers mit einer Primärseite 24 und einer Sekundärseite 13 an einen Regelverstärker 14 zur Ansteuerung des Leistungsgenerators 11 übermittelt. Der Regelverstärker 14 vergleicht die Eingangsgröße, das heißt die mittels des Messverstärker 23 gemessene Messgröße mit einem Sollwert und stellt ein Differenzsignal an seinem Ausgang zur Verfügung. Durch diese Anordnung ergibt sich eine geschlossene Regelschleife, die eine Messgröße der Last, wie beispielsweise die Spannung an der Last oder den Strom durch die Last konstant hält. Anstelle der zuvor beschriebenen Messgröße sind selbstverständlich auch mehrere Messgrößen zur Regelung heranziehbar. Entsprechend sind auch mehrere Signalübertrager notwendig. Alternativ kann ein Signalübertrager im Multiplexverfahren betrieben werden. Nachteilig an dieser Anordnung ist, dass ein zusätzlicher Signalübertrager benötigt wird.

Fig. 3 zeigt schematisch einen erfindungsgemäßen Drehübertrager. Im Leistungspfad befindet sich auf der Primärseite ein Leistungsgenerator 11 zur Erzeugung eines hochfrequenten Wechselsignals oder eines pulsierenden Gleichsignals, welches in die Primärseite 12 des Leistungsübertragers eingespeist wird. Zwischen dem Leistungsgenerator und der Primärseite ist eine Messeinrichtung 15 vorgesehen. Diese Messeinrichtung ermittelt wenigstens eine, vorteilhafterweise mehrere elektrische Kenngrößen des Signals. So kann beispielsweise der Strom in die Primärseite 12 des Leistungsübertragers, die Spannung an der Primärseite 12 des Leistungsübertragers, ein Phasenwinkel oder eine andere Größe ermittelt werden. Die eine oder mehrere der ermittelten Größen werden über einen Messverstärker 16 weiter verstärkt und einer Funktionseinheit 17 zugeführt. Diese stellt wiederum ein Stellsignal für den Leistungsgenerator 11 zur Verfügung. Erfindungsgemäß steuert die Funktionseinheit 17 in Abhängigkeit von den Messgrößen den Leistungsgenerator. Hierzu besitzt sie ein Funktionsmodell der Übertragungsfunktion der Last transformiert auf die Primärseite 12 des Leistungsübertragers. Dieses Funktionsmodell leitet die Übertragungsfunktion der Last in Abhängigkeit von verschiedenen gemessenen Parametern her. So könnte beispielsweise durch das Funktionsmodell die an der Last anliegende Spannung in Abhängigkeit von der durch die Messeinrichtung 15 gemessenen Spannungs- beziehungsweise Stromwerte abgeschätzt werden. Ebenso könnte auch der in die Last fließende Strom oder die von der Last aufgenommene Leistung abgeschätzt werden.

Auf der Lastseite wird eine Last 22 durch die Sekundärseite 21 des Leistungsübertragers gespeist.

### Bezugszeichenliste

- 1: Rotierendes Teil
- 2: Stationäres Teil
- 3: Drehübertrager
- 11: Leistungsgenerator
- 12: Leistungsübertrager (Primärseite)
- 13: Signalübertrager (Sekundärseite)
- 14: Regelverstärker
- 15: Messeinrichtung
- 16: Messverstärker
- 17: Funktionseinheit
- 21: Leistungsübertrager (Sekundärseite)
- 22: Last
- 23: Messverstärker
- 24: Signalübertrager (Primärseite)
- 101: Röntgenröhre
- 102: Röntgenstrahlung
- 103: Detektor
- 104: Patient
- 105: bidirektionale Verbindung
- 106: Auswerte- und Steuereinheit
- 107: Patientenliege

## Patentansprüche

1. Induktiver Drehübertrager zur berührungslosen Übertragung elektrischer Energie zwischen einem stationären und einem rotierenden Teil, umfassend einen Leistungsgenerator (11) zur Erzeugung einer Wechselspannung oder eines Wechselstromes,
einen induktiven Leistungsübertrager umfassend eine Primärseite (12) und eine Sekundärseite (21), wobei die Primärseite durch den Leistungsgenerator (11) gespeist wird und die Sekundärseite (21) zur Speisung einer Last (22) dient, wobei eine Messeinrichtung (15) vorgesehen ist, welche zumindest eine elektrische Kenngröße, wie eine Spannung, einen Strom, ein Phasenwinkel der von dem Leistungsgenerator (11) in die Primärseite (12) des Leistungsübertragers eingespeisten elektrischen Energie ermittelt, und
**dadurch gekennzeichnet, dass**
eine Funktionseinheit (17) vorgesehen ist, welche unter Zuhilfenahme der zumindest einen elektrischen Kenngröße den Leistungsgenerator (11) derart steuert, dass zumindest eine weitere elektrische Kenngröße an der Last (22), wie eine Spannung, einen Strom, ein Phasenwinkel näherungsweise konstant gehalten wird, wobei die Funktionseinheit (17) ein Modell zur Nachbildung der Übertragungsfunktion, das heißt des Zusammenhangs zwischen der beziehungsweise den elektrischen Kenngrößen an der Last (23) und der beziehungsweise den elektrischen Kenngrößen an der Primärseite (12) aufweist.

2. Vorrichtung nach Anspruch 1,
**dadurch gekennzeichnet, dass**
das Modell eine Kennlinie oder eine Kennlinienschar ist, welche entsprechend der Anzahl der von der Messeinrichtung (15) gelieferten Eingangsparameter und der an den Regelverstärker (16) abgegebenen Ausgangsparameter auch mehrdimensional sein kann.

3. Vorrichtung nach einem der vorhergehenden Ansprüche,
**dadurch gekennzeichnet, dass**
das Modell das Verhalten der Anordnung unter der Annahme eines vereinfachten Ersatzschaltbildes nachbildet.

4. Vorrichtung nach einem der vorhergehenden Ansprüche,
**dadurch gekennzeichnet, dass**
das Modell auf wenigstens einer durch Messung gewonnenen Übertragungskennliinie basiert.

5. Vorrichtung nach einem der vorhergehenden Ansprüche,
**dadurch gekennzeichnet, dass**
das Modell zumindest einen Bereich der Übertragungsfunktion als lineare Funktion durch eine Gerade annähert.

6. Vorrichtung nach einem der vorhergehenden Ansprüche,
**dadurch gekennzeichnet, dass**
das Modell eine Nachbildung der Übertragungsfunktion durch analoge, passive Bauteile aufweist.

7. Vorrichtung nach einem der vorhergehenden Ansprüche,
**dadurch gekennzeichnet, dass**
das Modell zumindest einen Bereich der Übertragungsfunktion mittels eines Widerstands- und Diodennetzwerks annähert.

8. Vorrichtung nach einem der vorhergehenden Ansprüche,
**dadurch gekennzeichnet, dass**
das Modell zumindest einen Bereich der Übertragungsfunktion mittels eines logarithmischen Verstärkers annähert.

9. Vorrichtung nach einem der vorhergehenden Ansprüche,
**dadurch gekennzeichnet, dass**
das Modell auch wenigstens eine mechanische Größe, wie der Abstand zwischen rotierendem Teil (1) und stationärem Teil (2), die Größe des Luftspalts zwischen der Primärseite (12) des Leistungsübertragers und der Sekundärseite (21) des Leistungsübertragers, oder einer Winkelposition zwischen rotierendem Teil (1) und stationärem Teil (2) einbezieht.

10. Vorrichtung nach einem der vorhergehenden Ansprüche,
**dadurch gekennzeichnet, dass**
die Funktionseinheit einen digitalen Rechner, vorzugsweise einen digitalen Signalprozessor aufweist.

11. Vorrichtung nach einem der vorhergehenden Ansprüche,
**dadurch gekennzeichnet, dass**
das Modell als mathematisches Modell in der Funktionseinheit (17) hinterlegt ist und die Übertragungsfunktion zur Laufzeit berechnet wird.

12. Vorrichtung nach einem der vorhergehenden Ansprüche,
**dadurch gekennzeichnet, dass**
das Modell als Tabelle in einem Speicher abgelegt ist.

13. Vorrichtung nach einem der vorhergehenden Ansprüche,
**dadurch gekennzeichnet, dass**
die Funktionseinheit (17) Mittel zum Einmessen beziehungsweise Einlernen der Modellparameter aufweist.

14. Computertomograph umfassend einen induktiven Drehübertragern nach einem der vorhergehenden Ansprüche.

## Claims

1. Inductive rotary joint for non-contacting transmission of electrical energy between a stationary and a rotating part, comprising:
a power generator (11) for generating an alternating voltage or an alternating current,
an inductive power transfer device comprising a primary side (12) and a secondary side (21), in which the primary side is fed by the power generator (11) and the secondary side (21) serves to feed a load (22), and in which a measuring means (15) is provided for determining at least one electrical parameter such as a voltage, a current, a phase angle of the electrical energy fed into primary side (12) of the power transfer device by the power generator (11), and
**characterized in that**
a functions unit (17) is provided for controlling the power generator (11) with the aid of the at least one electrical parameter so that at least one further electrical parameter at the load (22), such as a voltage, a current, a phase angle is maintained approximately constant, with the functions unit (17) comprising a model for simulating the transfer function, i.e. the relationship between the electrical parameter or parameters at the load (23) and the electrical parameter or parameters at the primary side (12).

2. Device according to claim 1,
**characterized in that**
the model is a characteristic or a family of characteristics which also can be multi-dimensional according to the number of input parameters supplied by the measurement means (15) and the output parameters supplied to the control amplifier (16).

3. Device according to any one of the preceding claims,
**characterized in that**
the model simulates the behaviour of the arrangement under the assumption of a simplified substitute circuit.

4. Device according to any one of the preceding claims,
**characterized in that**
the model is based upon at least one transmission characteristic obtained by measurement.

5. Device according to any one of the preceding claims,
**characterized in that**
the model approximates at least one region of the transfer function as a linear function by a straight line.

6. Device according to any one of the preceding claims,
**characterized in that**
the model comprises a simulation of the transfer function by analogue passive components.

7. Device according to any one of the preceding claims,
**characterized in that**
the model approximates at least one region of the transfer function by means of a resistor and diode network.

8. Device according to any one of the preceding claims,
**characterized in that**
the model approximates at least one region of the transfer function by means of a logarithmic amplifier.

9. Device according to any one of the preceding claims,
**characterized in that**
the model takes into account also at least one mechanical parameter such as the distance between the rotating part (1) and the stationary part (2), the size of the air-gap between the primary side (12) of the power transfer device and the secondary side (21) of the power transfer device, or an angular position between the rotating part (1) and the stationary part (2).

10. Device according to any one of the preceding claims,
**characterized in that**
the functions unit comprises a digital computer, preferably a digital signal processor.

11. Device according to any one of the preceding claims,
**characterized in that**
the model is deposited in the functions unit (17) as a mathematical model, and the transfer function is calculated at the time of operation.

12. Device according to any one of the preceding claims,
**characterized in that**
the model is deposited as a table in a memory.

13. Device according to any one of the preceding claims,
**characterized in that**
the functions unit (17) comprises means for measuring-in or teaching-in the model parameters.

14. Computer tomograph comprising an inductive rotary joint as set out in any one of the preceding claims.

## Revendications

1. Transmetteur rotatif à induction pour la transmission sans contact d'énergie électrique entre une partie stationnaire et une partie en rotation, comprenant un générateur de puissance (11) pour générer une tension alternative ou un courant alternatif, un transmetteur de puissance à induction comprenant un côté primaire (12) et un côté secondaire (21), le côté primaire étant alimenté par le générateur de puissance (11) et le côté secondaire (21) servant à alimenter une charge (22), dans lequel est prévu un dispositif de mesure (15) qui détermine au moins une grandeur caractéristique électrique telle qu'une tension, une intensité, un angle de phase de l'énergie électrique fournie par le générateur de puissance (11) au côté primaire (12) du transmetteur de puissance, et
**caractérisé en ce qu'**il est prévu une unité fonctionnelle (17) qui commande le générateur de puissance (11) en s'aidant de l'au moins une grandeur caractéristique électrique, de telle manière qu'au moins une autre grandeur caractéristique électrique au niveau de la charge (22), telle qu'une tension, une intensité, un angle de phase, soit maintenue approximativement constante, l'unité fonctionnelle (17) comportant un modèle pour reproduire la fonction de transmission, c'est-à-dire la relation entre la ou les grandeurs caractéristiques électriques au niveau de la charge (23) et la ou les grandeurs caractéristiques électriques sur le côté primaire (12).

2. Dispositif selon la revendication 1, **caractérisé en ce que** le modèle est une courbe caractéristique ou une famille de courbes caractéristiques qui peut également être multidimensionnelle, selon le nombre des paramètres d'entrée fournis par le dispositif de mesure (15) et des paramètres de sortie émis au niveau de l'amplificateur de réglage (16).

3. Dispositif selon l'une des revendications précédentes, **caractérisé en ce que** le modèle reproduit le comportement du dispositif dans l'hypothèse d'un schéma de connexions de substitution simplifié.

4. Dispositif selon l'une des revendications précédentes, **caractérisé en ce que** le modèle se base sur au moins une courbe caractéristique de transmission obtenue par la mesure.

5. Dispositif selon l'une des revendications précédentes, **caractérisé en ce que** le modèle se rapproche d'au moins une partie de la fonction de transmission par une courbe sous la forme d'une fonction linéaire.

6. Dispositif selon l'une des revendications précédentes, **caractérisé en ce que** le modèle comporte une représentation de la fonction de transmission par des composants analogiques passifs.

7. Dispositif selon l'une des revendications précédentes, **caractérisé en ce que** le modèle se rapproche d'au moins une partie de la fonction de transmission au moyen d'un réseau de résistances et de diodes.

8. Dispositif selon l'une des revendications précédentes, **caractérisé en ce que** le modèle se rapproche au moins d'une partie de la fonction de transmission au moyen d'un amplificateur logarithmique.

9. Dispositif selon l'une des revendications précédentes, **caractérisé en ce que** le modèle inclut aussi au moins une grandeur mécanique, telle que la distance entre la partie rotative (1) et la partie stationnaire (2), la taille de l'entrefer entre le côté primaire (12) du transmetteur de puissance et le côté secondaire (21) du transmetteur de puissance, ou une position angulaire entre la partie rotative (1) et la partie stationnaire (2).

10. Dispositif selon l'une des revendications précédentes, **caractérisé en ce que** l'unité fonctionnelle comporte un calculateur numérique, de préférence un processeur de signaux numérique.

11. Dispositif selon l'une des revendications précédentes, **caractérisé en ce que** le modèle est enregistré sous forme de modèle mathématique dans l'unité fonctionnelle (17) et la fonction de transmission est calculée par rapport au temps d'exécution.

12. Dispositif selon l'une des revendications précédentes, **caractérisé en ce que** le modèle est enregistré sous forme de table dans une mémoire.

13. Dispositif selon l'une des revendications précédentes, **caractérisé en ce que** l'unité fonctionnelle (17) présente des moyens pour acquérir par la mesure ou par l'apprentissage les paramètres du modèle.

14. Appareil de tomographie assistée par ordinateur comprenant un transmetteur rotatif à induction selon l'une des revendications précédentes.
